(19) Europäisches Patentamt
European Patent Office
Office européen des brevets

(11) EP 3 789 040 A1

(12) **EUROPEAN PATENT APPLICATION**
published in accordance with Art. 153(4) EPC

(43) Date of publication:
**10.03.2021 Bulletin 2021/10**

(21) Application number: **19793796.4**

(22) Date of filing: **26.04.2019**

(51) Int Cl.:
***A61K 45/00*** (2006.01) ***A61K 31/4985*** (2006.01)
***A61K 31/505*** (2006.01) ***A61K 31/506*** (2006.01)
***A61K 31/517*** (2006.01) ***A61K 31/519*** (2006.01)
***A61K 31/52*** (2006.01) ***A61K 31/522*** (2006.01)
***A61P 9/00*** (2006.01) ***A61P 13/12*** (2006.01)
***A61P 17/00*** (2006.01) ***A61P 37/06*** (2006.01)
***A61P 43/00*** (2006.01)

(86) International application number:
**PCT/JP2019/018054**

(87) International publication number:
**WO 2019/208805 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2018 JP 2018085972**

(71) Applicant: **ONO Pharmaceutical Co., Ltd.**
**Osaka-shi, Osaka 541-8526 (JP)**

(72) Inventor: **ARIZA, Yuko**
**Mishima-gun, Osaka 618-8585 (JP)**

(74) Representative: **Jones, Nicholas Andrew**
**Withers & Rogers LLP**
**4 More London Riverside**
**London, SE1 2AU (GB)**

(54) **PREVENTIVE AND/OR THERAPEUTIC AGENT FOR AUTOIMMUNE DISEASE COMPRISING COMPOUND HAVING BTK INHIBITORY ACTIVITY AS ACTIVE INGREDIENT**

(57) The problem of the present invention is to find an effective preventive and/or therapeutic agent for an autoimmune disease, in particular, pemphigus, pemphigoid, ANCA-related angiitis and to provide the same as a medicine. The compounds having a Btk inhibitory activity to be used in the present invention inhibits antibody production from B cells and, moreover, inhibits the formation of neutrophil extracellular traps (NETs). Thus, these compounds are useful for preventing and/or treating an autoimmune disease, in particular, pemphigus, pemphigoid, ANCA-related angiitis

EP 3 789 040 A1

## Description

[Technical Field]

The present invention relates, in an embodiment, to a preventive and/or therapeutic agent for autoimmune diseases, comprising as an active ingredient a compound having a Btk inhibitory activity, wherein the compound having a Btk inhibitory activity is, e.g., ibrutinib, acalabrutinib, tirabrutinib, evobrutinib, or a pharmaceutically acceptable salt of the preceding.

[Background Art]

Bruton's tyrosine kinase (abbreviated below as Btk) belongs to the Tec family of kinases, which are non-receptor tyrosine kinases, and is selectively expressed in cells in B cell and myeloid cell lines. Btk plays an important role in B cell signal transduction and is a factor that contributes to B cell survival, differentiation, proliferation, activation, and so forth. B cell signaling via the B-cell antigen receptor (BCR) induces a broad range of biological reactions, and abnormal signal transduction here causes, for example, abnormal B cell activation, the formation of pathogenic autoantibodies, and so forth. Btk is thought to be a link in BCR-mediated signal transduction pathways into the B cell. The compounds having a Btk inhibitory activity and targeting various autoimmune diseases have been developed in recent years, and, for example, it has been reported that a clinical trial is underway with evobrutinib in patients with rheumatoid arthritis or systemic lupus erythematosus (SLE) (refer to NPL 1) and that clinical trials are underway with PRN-1008 in patients with pemphigus or idiopathic thrombocytopenic purpura (refer to NPL 2 and NPL 3).

ANCA-associated vasculitis (AAV), which is one type of autoimmune disease, is a disease in which - due to, for example, an immune abnormality - autoantibodies or anti-neutrophil cytoplasmic antibodies (ANCA) are produced against neutrophils and angiitis is brought about at various locations. AAV is classified into systemic types, in which angiitis is produced in several organs in the body, and organ-limited types, in which angiitis is produced in only one organ. Systemic AAV encompasses three diseases, i.e., microscopic polyangiitis (MPA), granulomatosis with polyangiitis (GPA) (also known as Wegener's granulomatosis (WG)), and eosinophilic granulomatosis with polyangiitis (EGPA) (also known as Churg-Strauss syndrome (CSS)). Renal-limited vasculitis (RLV), in which vasculitis is produced only in the kidneys, is known as an organ-limited AAV.

It is known that an abnormality with neutrophil extracellular traps (NETs) participates as a factor in ANCA production (refer to NPL 4).

On the other hand, several patent applications relating to compounds having a Btk inhibitory activity have been published to date (refer, for example, to PTL 1 and PTL 2). However, the action of compounds having a Btk inhibitory activity on NETs was unknown, and the effect of compounds having a Btk inhibitory activity on ANCA-related angiitis was also unclear.

[Citation List]

[Patent Literature]

[PTL 1] WO 2011/152351
[PTL 2] WO 2013/081016

[Non Patent Literature]

[NPL 1] ABSTRACT NUMBER: 2565, ACR/ARHP Annual Meeting, 2017
[NPL 2] ClinicalTrials.gov Identifier: NCT02704429
[NPL 3] ClinicalTrials.gov Identifier: NCT03395210
[NPL 4] Japanese Journal of Pediatric Nephrology, Volume 27, Number 2, pp. 11-15, 2014

[Summary of Invention]

[Technical Problem]

The present invention addresses the problem of finding an effective preventive and/or therapeutic agent for autoimmune diseases and providing same as a medicine.

[Solution to Problem]

As a result of intensive investigations in order to solve this problem, the present inventors discovered that this problem can be solved by a drug that contains a compound having a Btk inhibitory activity as an active ingredient.
The present invention thus provides, as embodiments thereof, for example,

[1] a preventive and/or therapeutic agent for an autoimmune disease selected from the group consisting of pemphigus, pemphigoid, ANCA-related angiitis, IgG4-related disease, nephrotic syndrome, and cutaneous lupus erythematosus, containing a compound having a Btk inhibitory activity;
[2] the agent according to [1], wherein the autoimmune disease is pemphigus, pemphigoid, ANCA-related angiitis, or nephrotic syndrome;
[3] the agent according to [1] or [2], wherein the autoimmune disease is ANCA-related angiitis;
[4] the agent according to any one of [1] to [3], wherein the ANCA-related angiitis is at least one selection from the group consisting of microscopic polyangiitis, granulomatosis with polyangiitis (Wegener's granulomatosis), eosinophilic granulomatosis with polyangiitis (Chug-Strauss syndrome), and renal-limited vasculitis;
[5] the agent according to any one of [1] to [4], wherein the ANCA-related angiitis is at least one selection from the group consisting of microscopic polyangiitis, eosinophilic granulomatosis with polyangiitis (Chug-Strauss syndrome), and renal-limited vasculitis;
[6] the agent according to any one of [1] to [5], wherein the ANCA-related angiitis is an MPO-ANCA positive and/or PR3-ANCA positive ANCA-related angiitis;
[7] an agent that inhibits the formation of neutrophil extracellular traps (NETs), containing a compound having a Btk inhibitory activity;
[8] the agent according to any one of [1] to [7], wherein the compound having a Btk inhibitory activity is a compound represented by general formula (I):

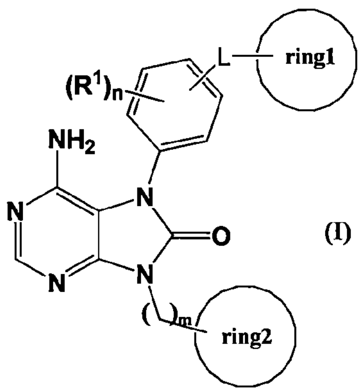

(I)

(in the formula, L represents (1) -O-, (2) -S-, (3) -SO-, (4) $-SO_2-$, (5) -NH-, (6) -C(O)-, (7) $-CH_2-O-$, (8) $-O-CH_2-$, (9) - $CH_2$-, or (10) -CH(OH)-;
$R^1$ represents (1) a halogen atom, (2) a $C_{1-4}$ alkyl group, (3) a $C_{1-4}$ alkoxy group, (4) a $C_{1-4}$ haloalkyl group, or (5) a $C_{1-4}$ haloalkoxy group;
ring1 represents a 4- to 7-membered cyclic group, which may be substituted by one to five substituents each independently selected from the group consisting of (1) halogen atoms, (2) $C_{1-4}$ alkyl groups, (3) $C_{1-4}$ alkoxy groups, (4) nitrile, (5) $C_{1-4}$ haloalkyl groups, and (6) $C_{1-4}$ haloalkoxy groups, provided that when two or more substituents are present on the ring1, these substituents may form a 4- to 7-membered cyclic group together with the atoms constituting ring1 to which these substituents are bonded;
ring2 represents a 4- to 7-membered saturated heterocycle, optionally substituted with one to three $-K-R^{2}$' s;
K represents (1) a bond, (2) a $C_{1-4}$ alkylene, (3) -C(O)-, (4) - $C(O)-CH_2-$, (5) $-CH_2-C(O)-$, (6) -C(O)O-, or (7) $-SO_2-$ (provided that the bonding site on the left bonds to the ring2);

$R^2$ represents (1) a $C_{1-4}$ alkyl, (2) a $C_{2-4}$ alkenyl, or (3) a $C_{2-4}$ alkynyl group, which may be substituted by one to five substituents each independently selected from the group consisting of (1) $NR^3R^4$, (2) halogen atoms, (3) $CONR^5R^6$, (4) $CO_2R^7$, and (5) $OR^8$;
$R^3$ and $R^4$ each independently represent (1) a hydrogen atom or (2) a $C_{1-4}$ alkyl group optionally substituted by $OR^9$ or $CONR^{10}R^{11}$ ;
$R^3$ and $R^4$ may form, together with the nitrogen atom to which they are bonded, a 4- to 7-membered nitrogenous saturated heterocycle optionally substituted by an oxo group or a hydroxyl group;
$R^5$ and $R^6$ each independently represent (1) a hydrogen atom, (2) a $C_{1-4}$ alkyl group, or (3) a phenyl group;
$R^7$ represents (1) a hydrogen atom or (2) a $C_{1-4}$ alkyl group;
$R^8$ represents (1) a hydrogen atom, (2) a $C_{1-4}$ alkyl group, (3) a phenyl group, or (4) a benzotriazolyl group;
$R^9$ represents (1) a hydrogen atom or (2) a $C_{1-4}$ alkyl group;
$R^{10}$ and $R^{11}$ each independently represent (1) a hydrogen atom or (2) a $C_{1-4}$ alkyl group;
n represents an integer from 0 to 4;
m represents an integer from 0 to 2; and
when n is two or more, the $R^{1}$' s may be the same or may differ from one another.)
or a salt thereof;

[9] the agent according to any one of [1] to [7], wherein the compound having a Btk inhibitory activity is at least one selected from the group consisting of tirabrutinib, ibrutinib, acalabrutinib, evobrutinib, fenebrutinib/GDC-0853, poseltinib/LY3337641, spebrutinib, vecabrutinib/SNS-062, zanubrutinib/BGB-3111, PRN1008, BMS-986142, branebrutinib/BMS-986195, LOU-064, M-7583, AC-058, DTRMWXHS-12, TAS-5315, TAK-020, ARQ-531, BMS-935177, PCI-45292, PRN-2246, SHR-1459, ABBV-105, CT-1530, ICP022, LOXO-305, JNJ-64264681, HWH-486, and salts thereof;
[10] the agent according to any one of [1] to [7] or [9], wherein the compound having a Btk inhibitory activity is at least one selection from the group consisting of tirabrutinib, ibrutinib, spebrutinib, acalabrutinib, evobrutinib, poseltinib, fenebrutinib, vecabrutinib, zanubrutinib, PRN-1008, BMS-986142, and salts thereof;
[11] the agent according to any one of [1] to [7] or [9] or [10], wherein the compound having a Btk inhibitory activity is at least one selection from the group consisting of tirabrutinib, ibrutinib, acalabrutinib, fenebrutinib, BMS-986142, evobrutinib, poseltinib, and salts thereof;
[12] the agent according to any one of [1] to [11], wherein the compound having a Btk inhibitory activity is tirabrutinib or a salt thereof;
[13] use of a compound having a Btk inhibitory activity to produce a preventive and/or therapeutic agent for an autoimmune disease selected from the group consisting of pemphigus, pemphigoid, ANCA-related angiitis, IgG4-related disease, nephrotic syndrome, and cutaneous lupus erythematosus;
[14] a method for preventing and/or treating an autoimmune disease selected from the group consisting of pemphigus, pemphigoid, ANCA-related angiitis, IgG4-related disease, nephrotic syndrome, and cutaneous lupus erythematosus, including administering an effective amount of a compound having a Btk inhibitory activity to a mammal;
[15] a compound having a Btk inhibitory activity, for use in the prevention and/or treatment of an autoimmune disease selected from the group consisting of pemphigus, pemphigoid, ANCA-related angiitis, IgG4-related disease, nephrotic syndrome, and cutaneous lupus erythematosus; and
[16] a preventive and/or therapeutic agent for an inflammatory · allergic disease selected from the group consisting of idiopathic urticaria, severe asthma, and eosinophilic sinusitis, comprising a compound having a Btk inhibitory activity.

[Advantageous Effects of Invention]

The present invention is useful for the prevention and/or treatment of autoimmune diseases.

[Description of Embodiments]

[Compounds Having a Btk Inhibitory Activity]

The compounds having a Btk inhibitory activity to be used in the present invention are, in one embodiment, low molecular weight compounds, and an embodiment thereof are the compounds represented by the following general formula (I) that are described in PTL 1 (WO 2011/152351)

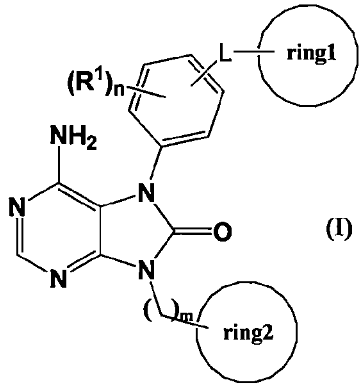

(I)

(Each of the variable groups (L, $R^1$, ring1, ring2, n, and m) in the formula have the same definitions as described in PTL 1.) and their optical isomers or mixtures, salts, solvates, N-oxides, or prodrugs.

The variable groups in general formula (I) specifically represent the following. That is, L represents (1) -O-, (2) -S-, (3) -SO-, (4) $-SO_2-$, (5) -NH-, (6) -C(O)-, (7) $-CH_2-O-$, (8) $-O-CH_2-$, (9) $-CH_2-$, or (10) -CH(OH)-; $R^1$ represents (1) a halogen atom, (2) a $C_{1-4}$ alkyl group, (3) a $C_{1-4}$ alkoxy group, (4) a $C_{1-4}$ haloalkyl group, or (5) a $C_{1-4}$ haloalkoxy group; ring1 represents a 4- to 7-membered cyclic group, which may be substituted by one to five substituents each independently selected from the group consisting of (1) halogen atoms, (2) $C_{1-4}$ alkyl groups, (3) $C_{1-4}$ alkoxy groups, (4) nitrile, (5) $C_{1-4}$ haloalkyl groups, and (6) $C_{1-4}$ haloalkoxy groups, wherein when two or more substituents are present on the ring1, these substituents may form a 4- to 7-membered cyclic group together with the atoms constituting ring1 to which these substituents are bonded; ring2 represents a 4- to 7-membered saturated heterocycle, optionally substituted with one to three $-K-R^{2}$' s; K represents (1) a bond, (2) a $C_{1-4}$ alkylene, (3) -C(O)-, (4) $-C(O)-CH_2-$, (5) $-CH_2-C(O)-$, (6) -C(O)O-, or (7) $-SO_2-$ (wherein the bond on the left is the bond to the ring2); $R^2$ represents (1) a $C_{1-4}$ alkyl, (2) a $C_{2-4}$ alkenyl, or (3) a $C_{2-4}$ alkynyl group, which may be substituted by one to five substituents each independently selected from the group consisting of (1) $NR^3R^4$, (2) halogen atoms, (3) $CONR^5R^6$, (4) $CO_2R^7$, and (5) $OR^8$; $R^3$ and $R^4$ each independently represent (1) a hydrogen atom or (2) a $C_{1-4}$ alkyl group optionally substituted by $OR^9$ or $CONR^{10}R^{11}$; $R^3$ and $R^4$ may form, together with the nitrogen atom to which they are bonded, a 4-to 7-membered nitrogenous saturated heterocycle optionally substituted by an oxo group or a hydroxyl group; $R^5$ and $R^6$ each independently represent (1) a hydrogen atom, (2) a $C_{1-4}$ alkyl group, or (3) a phenyl group; $R^7$ represents (1) a hydrogen atom or (2) a $C_{1-4}$ alkyl group; $R^8$ represents (1) a hydrogen atom, (2) a $C_{1-4}$ alkyl group, (3) a phenyl group, or (4) a benzotriazolyl group; $R^9$ represents (1) a hydrogen atom or (2) a $C_{1-4}$ alkyl group; $R^{10}$ and $R^{11}$ each independently represent (1) a hydrogen atom or (2) a $C_{1-4}$ alkyl group; n represents an integer from 0 to 4; m represents an integer from 0 to 2; and when n is two or more, the $R^{1}$' s may be the same or may differ from one another.

The halogen atom in the present invention denotes fluorine, chlorine, bromine, and iodine.

The $C_{1-4}$ alkyl group in the present invention denotes straight-chain and branched-chain $C_{1-4}$ alkyl groups such as methyl, ethyl, propyl, isopropyl, butyl, sec-butyl, and tert-butyl.

The $C_{1-4}$ alkylene group in the present invention denotes, for example, methylene, ethylene, propylene, and butylene and their isomers.

The $C_{1-4}$ alkoxy group in the present invention denotes straight-chain and branched-chain $C_{1-4}$ alkoxy groups such as methoxy, ethoxy, propoxy, isopropoxy, butoxy, isobutyloxy, and tert-butoxy.

The $C_{2-4}$ alkenyl group in the present invention denotes straight-chain and branched-chain $C_{2-4}$ alkenyl groups such as ethenyl, 1-propenyl, 2-propenyl, 1-butenyl, 2-butenyl, 3-butenyl, and 1,3-butadienyl.

The $C_{2-4}$ alkynyl group in the present invention denotes straight-chain and branched-chain $C_{2-4}$ alkynyl groups such as ethynyl, 1-propynyl, 2-propynyl, 1-butynyl, 2-butynyl, 3-butynyl, and 1,3-butadiynyl.

The $C_{1-4}$ haloalkyl group in the present invention denotes a group provided by the substitution of one or two or more halogen atoms onto a $C_{1-4}$ alkyl group, for example, the fluoromethyl group, chloromethyl group, bromomethyl group, iodomethyl group, difluoromethyl group, trifluoromethyl group, 1-fluoroethyl group, 2-fluoroethyl group, 2-chloroethyl group, pentafluoroethyl group, 1-fluoropropyl group, 2-chloropropyl group, 3-fluoropropyl group, 3-chloropropyl group, 4,4,4-trifluorobutyl group, and 4-bromobutyl group.

The $C_{1-4}$ haloalkoxy in the present invention denotes a group provided by the substitution of one or two or more halogen atoms onto a $C_{1-4}$ alkoxy group, for example, the trifluoromethoxy group, trichloromethoxy group, chloromethoxy group, bromomethoxy group, fluoromethoxy group, iodomethoxy group, difluoromethoxy group, dibromomethoxy group, 2-chloroethoxy group, 2,2,2-trifluoroethoxy group, 2,2,2-trichloroethoxy group, 3-bromopropoxy group, 3-chloropropoxy group, 2,3-dichloropropoxy group, 1-fluorobutoxy group, 4-fluorobutoxy group, and 1-chlorobutoxy group.

The 4- to 7-membered cyclic group in the present invention denotes a $C_{4-7}$ carbocycle or a 4- to 7-membered

heterocycle.

The $C_{4-7}$ carbocycle in the present invention denotes a $C_{4-7}$ monocyclic aliphatic or aromatic carbocycle. The aliphatic system may be partially or completely saturated. Examples are cyclobutane, cyclopentane, cyclohexane, cycloheptane, cyclopentene, cyclohexene, cycloheptene, cyclobutadiene, cyclopentadiene, cyclohexadiene, cycloheptadiene, and benzene.

The 4- to 7-membered heterocycle in the present invention denotes a 4- to 7-membered unsaturated heterocycle or a 4- to 7-membered saturated heterocycle.

The 4- to 7-membered unsaturated heterocycle in the present invention denotes an unsaturated 4- to 7-membered monocyclic heterocycle that contains from one to five heteroatoms selected from the oxygen atom, nitrogen atom, and sulfur atom, and can be exemplified by pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, and thiadiazepine.

The 4- to 7-membered saturated heterocycle in the present invention denotes a partially or fully saturated 4- to 7-membered monocyclic heterocycle that contains from one to five heteroatoms each independently selected from the oxygen atom, nitrogen atom, and sulfur atom, and can be exemplified by azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, oxetane, dihydrofuran, tetrahydrofuran, dihydropyran, tetrahydropyran, dihydrooxepine, tetrahydrooxepine, perhydrooxepine, thietane, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, dioxolane, dioxane, dithiolane, and dithiane.

The 4- to 7-membered nitrogenous saturated heterocycle in the present invention refers to a 4- to 7-membered saturated heterocycle that necessarily contains one or more nitrogen atoms. Examples are azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, and thiomorpholine.

A preferred embodiment among the compounds described in PTL 1 is tirabrutinib (CAS Registry Number: 1351636-18-4)(also referred to as 6-amino-9-[(3R)-1-(buta-2-ynoyl)pyrrolidin-3-yl]-7-(4-phenoxyphenyl)-7,9-dihydro-8H-purin-8-one), which is described in Example 19(2) and has the structure given below, or a salt thereof.

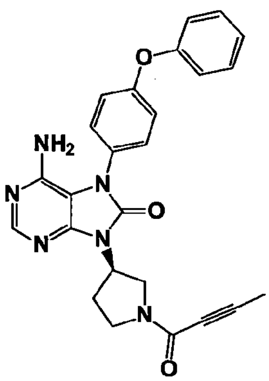

Tirabrutinib hydrochloride (CAS Registry Number: 1439901-97-9) is a particularly preferred embodiment.

The following are examples of other embodiments of compounds having a Btk inhibitory activity to be used in the present invention: compounds described in the examples in, e.g., WO 2008/039218, WO 2008/121742, WO 2013/010868, WO 2012/170976, WO 2013/067274, WO 2011/162515, WO 2009/158571, WO 2013/185084, WO 2014/173289, WO 2014/039899, WO 2014/210085, and WO 2016/065226. Embodiments at a more specific level are ibrutinib (CAS Registry Number: 936563-96-1), acalabrutinib (CAS Registry Number: 1420477-60-6), evobrutinib/M2951 (CAS Registry Number: 1415823-73-2), fenebrutinib/GDC-0853 (CAS Registry Number: 1434048-34-6), poseltinib/LY3337641 (CAS Registry Number: 1353552-97-2), spebrutinib (CAS Registry Number: 1202757-89-8), vecabrutinib/SNS-062 (CAS Registry Number: 1510829-06-7), zanubrutinib/BGB-3111 (CAS Registry Number: 1691249-45-2), PRN1008, BMS-986142, branebrutinib/BMS-986195, LOU-064, M-7583, AC-058, DTRMWXHS-12, TAS-5315, TAK-020, ARQ-531, BMS-935177, PCI-45292, PRN-2246, SHR-1459, ABBV-105, CT-1530, ICP022, LOXO-305, JNJ-64264681, and HWH-486, or their salts.

The present invention encompasses all isomers unless specifically indicated otherwise. For example, both straight-chain alkyl groups and branched-chain alkyl groups are encompassed by the alkyl groups. All of the following are also encompassed by the present invention: geometric isomers (E configuration, Z configuration, cis configuration, trans configuration) for double bonds, rings, and condensed rings; optical isomers due to, for example, the presence of an asymmetric carbon atom (R and S configurations, $\alpha$ and $\beta$ positions, enantiomers, diastereomers); optically active forms that exhibit optical rotation (D, L, d, and 1 configurations); polar forms generated by chromatographic separation (high-polarity forms, low-polarity forms); equilibrium compounds; rotational isomers; mixtures of the preceding in any proportions; and racemic mixtures. The present invention also encompasses all isomers due to tautomers.

In addition, an optical isomer for the compounds having a Btk inhibitory activity to be used in the present invention refers not only to the 100% pure optical isomer, but may also include less than 50% of another optical isomer.

The compounds having a Btk inhibitory activity to be used in the present invention may be converted into salts using known methods. These salts are preferably pharmaceutically acceptable salts and are also preferably water-soluble salts. Suitable pharmaceutically acceptable salts can be exemplified by salts with alkali metals (potassium, sodium, and so forth), salts with alkaline-earth metals (calcium, magnesium, and so forth), the ammonium salt, salts with pharmaceutically acceptable organic amines (tetramethylammonium, triethylamine, methylamine, dimethylamine, cyclopentylamine, benzylamine, phenethylamine, piperidine, monoethanolamine, diethanolamine, tris(hydroxymethyl)aminomethane, lysine, arginine, N-methyl-D-glucamine, and so forth), and acid addition salts (inorganic acid salts (hydrochloride, hydrobromide, hydroiodide, sulfate, phosphate, nitrate, and so forth) and organic acid salts (acetate, trifluoroacetate, lactate, tartrate, oxalate, fumarate, maleate, benzoate, citrate, methanesulfonate, ethanesulfonate, benzenesulfonate, toluenesulfonate, isethionate, glucuronate, gluconate, and so forth)).

The compounds having a Btk inhibitory activity to be used in the present invention, or salt thereof, may also be converted into a hydrate.

The compounds having a Btk inhibitory activity to be used in the present invention may be labeled with, for example, an isotope (for example, $^{2}H$, $^{3}H$, $^{11}C$, $^{13}C$, $^{14}C$, $^{13}N$, $^{15}N$, $^{15}O$, $^{17}O$, $^{18}O$, $^{35}S$, $^{18}F$, $^{36}Cl$, $^{123}I$, $^{125}I$, and so forth).

The compounds having a Btk inhibitory activity to be used in the present invention can be produced according to the examples described in the various patent documents. For example, tirabrutinib can be produced according to Example 19(2) in PTL 1.

The compounds having a Btk inhibitory activity to be used in the present invention are generally administered systemically or locally in an oral or parenteral form. The oral formulations can be exemplified by liquids for oral administration (for example, elixirs, syrups, pharmaceutically acceptable water-based formulations, suspensions, and emulsions) and solids for oral administration (for example, tablets (including sublingual tablets and orally disintegrating tablets), pills, capsules (including hard capsules, soft capsules, gelatin capsules, and microcapsules), powders, granules, and

lozenges). The parenteral formulations can be exemplified by solutions (for example, injectables (for example, subcutaneous injectables, intravenous injectables, intramuscular injectables, intraperitoneal injectables, and drip formulations), eye drops (for example, aqueous eye drop formulations (for example, aqueous eye drops, aqueous eye drop suspensions, viscous eye drops, and solubilized eye drops) and nonaqueous eye drop formulations (for example, nonaqueous eye drops and nonaqueous eye drop suspensions))), topicals (for example, ointments (for example, ophthalmic ointments)), and ear drops. These formulations may be controlled release formulations such as rapid release formulations, sustained release formulations, and so forth. These formulations can be produced by known methods, for example, by the methods described in The Japanese Pharmacopoeia.

The liquids for oral administration as the oral formulations can be produced, for example, by dissolving, suspending, or emulsifying the compound having a Btk inhibitory activity to be used in the present invention, in a commonly used diluent (for example, purified water, ethanol, or a mixture thereof). These liquid formulations may also contain, for example, a wetting agent, suspending agent, emulsifying agent, sweetener, flavorant, fragrance, preservative, buffer, and so forth.

The solids for oral administration as the oral formulations are prepared, for example, by mixing the compound having a Btk inhibitory activity to be used in the present invention, with a excipient (for example, lactose, mannitol, glucose, microcrystalline cellulose, and starch), a binder (for example, hydroxypropyl cellulose, polyvinylpyrrolidone, and magnesium metasilicate aluminate), a disintegrant (for example, cellulose calcium glycolate), a lubricant (for example, magnesium stearate), a stabilizer, a dissolution adjuvant (for example, glutamic acid and aspartic acid), and so forth, and formulating according to common methods. As necessary, coating may be carried out with a coating agent (for example, sucrose, gelatin, hydroxypropyl cellulose, and hydroxypropyl methyl cellulose phthalate), and two or more layers may be applied.

Topicals as parenteral formulations may be produced using a known method or a formulation in common use. For example, an ointment may be produced by incorporating or melting the compound having a Btk inhibitory activity to be used in the present invention, into a base. The ointment base is selected from known ointment bases or an ointment base in common use. For example, a single selection from the following or a mixture of two or more selections from the following may be used: higher fatty acids and higher fatty acid esters (for example, adipic acid, myristic acid, palmitic acid, stearic acid, oleic acid, adipate esters, myristate esters, palmitate esters, stearate esters, and oleate esters), waxes (for example, beeswax, spermaceti, and ceresin), surfactants (for example, polyoxyethylene alkyl ether phosphate esters), higher alcohols (for example, cetanol, stearyl alcohol, and cetostearyl alcohol), silicone oils (for example, dimethylpolysiloxane), hydrocarbons (for example, hydrophilic petrolatum, white petrolatum, purified lanolin, and liquid paraffin), glycols (for example, ethylene glycol, diethylene glycol, propylene glycol, polyethylene glycol, and macrogol), plant oils (for example, castor oil, olive oil, sesame oil, and turpentine oil), animal oils (for example, mink oil, egg yolk oil, squalane, and squalene), water, absorption promoters, and anti-irritants. A humectant, preservative, stabilizer, antioxidant, fragrance, and so forth may also be incorporated.

Injectables as parenteral formulations encompass solutions, suspensions, and emulsions as well as solid injectables used by dissolution or suspension in a solvent at the time of use. For example, an injectable may be used in which the compound having a Btk inhibitory activity to be used in the present invention is dissolved, suspended, or emulsified in a solvent. For example, distilled water for injection, physiological saline solution, a plant oil, propylene glycol, polyethylene glycol, an alcohol such as ethanol, or a combination of the preceding may be used for the solvent. The injectable may also contain a stabilizer, a dissolution adjuvant (for example, glutamic acid, aspartic acid, and Polysorbate 80 (registered trademark)), a suspending agent, an emulsifying agent, a soothing agent, a buffer, a preservative, and so forth. The injectable may be sterilized in the final step or may be manufactured using aseptic processing. The injectable may also be manufactured as a sterile solid form, for example, a freeze-dried product, and may be used after dissolution in distilled water for injection or another solvent, which is either sterile or sterilized prior to use.

While this will vary depending on, inter alia, age, body weight, symptoms, therapeutic effect, method of administration, and treatment time, a compound having a Btk inhibitory activity to be used in the present invention may be orally administered to an adult at from one administration to several administrations per day in the range from 1 ng to 1,000 mg per one administration, or may be parenterally administered to an adult at from one administration to several administrations per day in the range from 0.1 ng to 100 mg per one administration, or may be continuously intravenously administered in the range from 1 hour to 24 hours per day. Because the dose will vary depending on a variety of conditions as noted above, there will certainly be acceptable instances in which the amount is less than the doses described above as well as instances that require administration in excess of the ranges.

[Toxicity]

The compounds having a Btk inhibitory activity to be used in the present invention exhibit a satisfactorily low toxicity and can be safely used as medicines.

[Applications to Medicines]

Compounds having a Btk inhibitory activity to be used in the present invention have, for example, a Btk inhibitory activity, an inhibitory action on antibody production from B cells, and so forth, and as a consequence can be used as a preventive and/or therapeutic agent for autoimmune diseases or inflammatory · allergic diseases in mammals and particularly humans.

Pemphigus, pemphigoid, ANCA-related angiitis, IgG4-related disease, nephrotic syndrome, and cutaneous lupus erythematosus are examples of embodiments of autoimmune diseases for the present invention. Pemphigus, pemphigoid, ANCA-related angiitis, and nephrotic syndrome are examples of preferred embodiments of autoimmune diseases, and ANCA-related angiitis is an example of a particularly preferred embodiment.

Idiopathic urticaria, severe asthma, and eosinophilic sinusitis are examples of embodiments of inflammatory · allergic diseases for the present invention.

Pemphigus vulgaris, pemphigus foliaceus, paraneoplastic pemphigus, pemphigus vegetans, pemphigus erythematosus, herpetiform pemphigus, and drug-induced pemphigus are examples of embodiments of pemphigus in the present invention.

Bullous pemphigoid, mucous membrane pemphigoid, and epidermolysis bullosa acquisita are examples of embodiments of pemphigoid in the present invention.

Microscopic polyangiitis (MPA), granulomatosis with polyangiitis (GPA) (also known as Wegener's granulomatosis (WG)), eosinophilic granulomatosis with polyangiitis (EGPA) (also known as Churg-Strauss syndrome (CSS)), and renal-limited vasculitis (RLV) are examples of embodiments of ANCA-related angiitis in the present invention. Microscopic polyangiitis, eosinophilic granulomatosis with polyangiitis, and renal-limited vasculitis are examples of preferred embodiments.

Autoimmune pancreatitis, IgG4-related dacryoadenitis, sialoadenitis (Mikulicz's disease), IgG4-related sclerosing cholangitis, IgG4-related kidney disease, IgG4-related ophthalmic disease, and IgG4-related respiratory disease are examples of embodiments of the IgG4-related disease in the present invention.

Primary nephrotic syndrome and secondary nephrotic syndrome are examples of the nephrotic syndrome in the present invention. Minimal change nephrotic syndrome (MCNS), focal segmental glomerulosclerosis (FSGS), membranous nephropathy (MN), and proliferative glomerulonephritis (divided into mesangial proliferative type, intraductal proliferative type, membranous proliferative type, and crescentic type) are examples of embodiments of primary nephrotic syndrome in the present invention. In addition, embodiments of secondary nephrotic syndrome refer to diseases caused by, for example, autoimmune diseases, metabolic diseases, infectious diseases, allergy · hypersensitivity diseases, tumors, drugs, and hereditary diseases.

The primary nephrotic syndrome in the present invention also includes pediatric primary nephrotic syndrome.

Neutrophil extracellular traps (NETs) participate in pathogenesis in ANCA-related angiitis. The compounds having a Btk inhibitory activity to be used in the present invention inhibit the formation of NETs and due to this can be used as a preventive and/or therapeutic agent for ANCA-related angiitis, for example, myeloperoxidase (MPO)-positive ANCA-related angiitis and proteinase3 (PR3)-positive ANCA-related angiitis.

The compound having a Btk inhibitory activity to be used in the present invention may also be administered in combination with another drug in order to (1) supplement and/or enhance the therapeutic effect of the compound, (2) improve the kinetics · absorption and/or reduce the dose, and/or (3) reduce adverse reactions.

The combination of another drug with the compound having a Btk inhibitory activity to be used in the present invention may be administered in the form of a compounded agent in which both components have been compounded into a single formulation, or may be administered in the form of separate formulations. Administration as separate formulations includes simultaneous administration and administration at different times. In the case of administration at different times, the compound having a Btk inhibitory activity to be used in the present invention may be administered first followed by administration of the other drug, or the other drug may be administered first followed by administration of the compound having a Btk inhibitory activity to be used in the present invention. The same method of administration may be used for each, or different methods of administration may be used.

There are no particular limitations on this combination, but the other drug should be able to supplement and/or enhance the preventive and/or therapeutic effect for autoimmune diseases or inflammatory · allergic diseases of the compound having a Btk inhibitory activity to be used in the present invention.

Steroids, immunosuppressants, anti-CD20 antibodies, and gammaglobulin are examples of an additional drug for supplementing and/or enhancing the preventive and/or therapeutic effect for pemphigus of the compounds having a Btk inhibitory activity to be used in the present invention.

The combination of a tetracycline-type antibiotic and nicotinamide, Steroids, immunosuppressants, anti-CD20 antibodies, and gammaglobulin are examples of an additional drug for supplementing and/or enhancing the preventive and/or therapeutic effect for pemphigoid of the compounds having a Btk inhibitory activity to be used in the present invention.

Steroids, immunosuppressants, anti-CD20 antibodies, gammaglobulin, anti-TNF-α agents, antithymocyte globulin (ATG), anti-CD52 antibodies, and C5a receptor antagonists are examples of an additional drug for supplementing and/or enhancing the preventive and/or therapeutic effect for ANCA-related angiitis of the compounds having a Btk inhibitory activity to be used in the present invention.

Steroids, immunosuppressants, anti-CD20 antibodies, angiotensin-converting enzyme (ACE) inhibitors, and angiotensin II receptor blockers (ARBs) are examples of an additional drug for supplementing and/or enhancing the preventive and/or therapeutic effect for nephrotic syndrome of the compounds having a Btk inhibitory activity to be used in the present invention.

The steroids can be exemplified by amcinonide, hydrocortisone sodium succinate, prednisolone sodium succinate, methylprednisolone sodium succinate, ciclesonide, difluprednate, betamethasone propionate, dexamethasone, deflazacort, triamcinolone, triamcinolone acetonide, halcinonide, dexamethasone palmitate, hydrocortisone, flumetasone pivalate, prednisolone butyl acetate, budesonide, prasterone sulfate, mometasone furoate, fluocinonide, fluocinolone acetonide, fludroxycortide, flunisolide, prednisolone, alclometasone propionate, clobetasol propionate, dexamethasone propionate, deprodone propionate, fluticasone propionate, beclometasone propionate, betamethasone, methylprednisolone, methylprednisolone suleptanate, methylprednisolone sodium succinate, dexamethasone sodium phosphate, hydrocortisone sodium phosphate, prednisolone sodium phosphate, diflucortolone valerate, dexamethasone valerate, betamethasone valerate, prednisolone valerate acetate, cortisone acetate, diflorasone acetate, dexamethasone acetate, triamcinolone acetate, paramethasone acetate, halopredone acetate, fludrocortisone acetate, prednisolone acetate, methylprednisolone acetate, clobetasone butyrate, hydrocortisone butyrate, hydrocortisone butyrate propionate, and betamethasone butyrate propionate.

The immunosuppressants can be exemplified by azathioprine, ascomycin, everolimus, salazosulfapyridine, cyclosporine, cyclophosphamide, sirolimus, tacrosimus, bucillamine, methotrexate, leflunomide, mizoribine, mycophenolate mofetil (MMF), dapsone, and 15-deoxyspergualin (DSG).

The anti-CD20 antibodies can be exemplified by rituximab, ibritumomab, ocrelizumab, ofatuzumab, and obinutuzumab.

The tetracycline-type antibiotics can be exemplified by tetracycline, minocycline, and doxycycline.

The anti-TNF-α agents can be exemplified by anti-TNF-α antibodies, soluble TNF-α receptor, anti-TNF-α receptor antibodies, and soluble TNF-α binding protein and particularly by infliximab and etanercept.

The anti-CD52 antibodies can be exemplified by alemtuzumab.

The C5a receptor antagonists can be exemplified by avacopan.

The angiotensin-converting enzyme (ACE) inhibitors can be exemplified by alacepril, imidapril hydrochloride, quinapril hydrochloride, temocapril hydrochloride, delapril hydrochloride, benazepril hydrochloride, captopril, trandolapril, perindopril erbumine, enalapril maleate, lisinopril hydrate, and cilazapril hydrate.

The angiotensin II receptor blockers can be exemplified by losartan (potassium), candesartan (cilexetil), valsartan, irbesartan, olmesartan (medoxomil), telmisartan, and azilsartan.

## Examples

The present invention is specifically described through the examples provided below, but the present invention is in no way limited to or by these.

### Biological Example 1: Measurement of Activation of Human Neutrophils

Human serum albumin (HSA) (Sigma) was immobilized on a 96-well plate and anti-HSA antibody (Sigma) was added to form an immune complex. Blocking was performed with 10% fetal bovine serum (FBS), and the plate was then used for neutrophil activation.

Various compounds having a Btk inhibitory activity were used as the test compounds. The test compound was dissolved in DMSO and dilution with RPMI 1640 culture medium (10% FBS, contained 1% penicillin/streptomycin) was then performed to prepare a test compound solution with a concentration 11-times that of the final concentration. 180 μL of a culture medium-diluted human neutrophil suspension was added to 20 μL of the 11X-concentration test compound solution, or the medium (1.1% DMSO), and incubation was performed for 1 hour at room temperature.

20 μL of a 50 μM SYTOX Green solution (Thermo Fisher Scientific Inc.) was then added to the plate bearing the immobilized immune complex; 180 μL ($1 \times 10^5$ cells/well) of the test compound-treated neutrophil suspension was added; and incubation was carried out for 4 hours at 37°C and 5% $CO_2$. The relative fluorescence units of the SYTOX Green bound to NETs from the neutrophils were measured using a microplate reader (Molecular Devices) and conditions of an excitation wavelength of 475 nm and a fluorescence wavelength of 523 nm. The percent inhibition (%) for the test compound was calculated using the formula given below. The percent inhibition (%) was graphically plotted using Prism (ver. 5.04, GraphPad Software) and the IC50 value was determined.

[Math. 1]

$$\text{Inhibition rate}(\%) = \left[ 1 - \frac{(A - C)}{(B - C)} \right] \times 100$$

A : relative fluorescence units when the test compound was added
B : relative fluorescence units at the time of stimulation
C : relative fluorescence units without stimulation

The IC50 value for the individual test compounds for NETs formation by the neutrophils was, for example, 0.022 μM for tirabrutinib. In addition, the IC50 values for ibrutinib, acalabrutinib, fenebrutinib, BMS-986142 (the compound of Example 28 in WO 2014/210085), evobrutinib, and the compound of Example 1 in WO 2011/162515 were, respectively, 0.0036 μM, 0.084 μM, 0.062 μM, 0.044 μM, 0.057 μM, and 0.285 μM. It was shown that all of these compounds having a Btk inhibitory activity had the ability to inhibit neutrophil activation, i.e., to inhibit the formation of NETs.

Biological Example 2: Evaluation of Effectiveness Using the Anti-GBM Antibody-Induced Nephritis Model

The efficacy of the individual test compounds in treating nephrotic syndrome can be evaluated using a nephritis model induced using an anti-glomerular basement membrane (GBM) antibody. As will be clear to the person skilled in the art, this anti-GBM antibody-induced nephritis model can be constructed using the experimental methods described in, for example, J. Immunol., 183: 3980-3988 (2009) and J. Immunol., 191: 4540-4550 (2013).
The individual test compounds is able to inhibit anti-GBM antibody-induced nephritis.

[Industrial Applicability]

The compounds having a Btk inhibitory activity to be used in the present invention are useful as preventive and/or therapeutic agents for autoimmune diseases and particularly ANCA-related angiitis.

## Claims

1. A preventive and/or therapeutic agent for an autoimmune disease selected from the group consisting of pemphigus, pemphigoid, ANCA-related angiitis, IgG4-related disease, nephrotic syndrome, and cutaneous lupus erythematosus, comprising a compound having a Btk inhibitory activity.

2. The agent according to claim 1, wherein the autoimmune disease is ANCA-related angiitis.

3. The agent according to claim 1 or 2, wherein the ANCA-related angiitis is at least one selection from the group consisting of microscopic polyangiitis, granulomatosis with polyangiitis (Wegener's granulomatosis), eosinophilic granulomatosis with polyangiitis (Chug-Strauss syndrome), and renal-limited vasculitis.

4. The agent according to any one of claims 1 to 3, wherein the ANCA-related angiitis is at least one selection from the group consisting of microscopic polyangiitis, eosinophilic granulomatosis with polyangiitis (Chug-Strauss syndrome), and renal-limited vasculitis.

5. The agent according to any one of claims 1 to 4, wherein the ANCA-related angiitis is an MPO-ANCA positive and/or PR3-ANCA positive ANCA-related angiitis.

6. An agent that inhibits the formation of neutrophil extracellular traps (NETs), comprising a compound having a Btk inhibitory activity.

7. The agent according to any one of claims 1 to 6, wherein the compound having a Btk inhibitory activity is a compound represented by general formula (I)

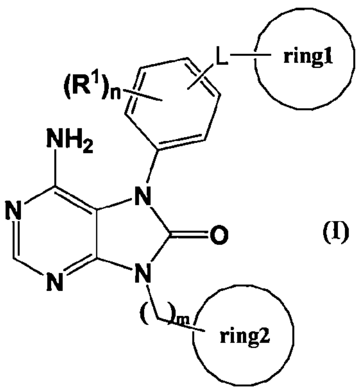

(I)

(in the formula, L represents (1) -O-, (2) -S-, (3) -SO-, (4) $-SO_2-$, (5) -NH-, (6) -C(O)-, (7) $-CH_2-O-$, (8) $-O-CH_2-$, (9) $-CH_2-$, or (10) -CH(OH)-;
$R^1$ represents (1) a halogen atom, (2) a $C_{1-4}$ alkyl group, (3) a $C_{1-4}$ alkoxy group, (4) a $C_{1-4}$ haloalkyl group, or (5) a $C_{1-4}$ haloalkoxy group;
ring1 represents a 4- to 7-membered cyclic group which may be substituted by one to five substituents each independently selected from the group consisting of (1) halogen atoms, (2) $C_{1-4}$ alkyl groups, (3) $C_{1-4}$ alkoxy groups, (4) nitrile, (5) $C_{1-4}$ haloalkyl groups, and (6) $C_{1-4}$ haloalkoxy groups, provided that when two or more substituents are present on the ring1, these substituents may form a 4- to 7-membered cyclic group together with the atoms constituting ring1 to which these substituents are bonded;
ring2 represents a 4- to 7-membered saturated heterocycle, optionally substituted with one to three $-K-R^2$'s;
K represents (1) a bond, (2) a $C_{1-4}$ alkylene, (3) -C(O)-, (4) - $C(O)-CH_2-$, (5) $-CH_2-C(O)-$, (6) -C(O)O-, or (7) $-SO_2-$ (provided that the bonding site on the left bonds to the ring2);
$R^2$ represents (1) a $C_{1-4}$ alkyl, (2) a $C_{2-4}$ alkenyl, or (3) a $C_{2-4}$ alkynyl group which may be substituted by one to five substituents each independently selected from the group consisting of (1) $NR^3R^4$, (2) halogen atoms, (3) $CONR^5R^6$, (4) $CO_2R^7$, and (5) $OR^8$;
$R^3$ and $R^4$ each independently represent (1) a hydrogen atom or (2) a $C_{1-4}$ alkyl group optionally substituted by $OR^9$ or $CONR^{10}R^{11}$;
$R^3$ and $R^4$ may form, together with the nitrogen atom to which they are bonded, a 4- to 7-membered nitrogenous saturated heterocycle optionally substituted by an oxo group or a hydroxyl group;
$R^5$ and $R^6$ each independently represent (1) a hydrogen atom, (2) a $C_{1-4}$ alkyl group, or (3) a phenyl group;
$R^7$ represents (1) a hydrogen atom or (2) a $C_{1-4}$ alkyl group;
$R^8$ represents (1) a hydrogen atom, (2) a $C_{1-4}$ alkyl group, (3) a phenyl group, or (4) a benzotriazolyl group;
$R^9$ represents (1) a hydrogen atom or (2) a $C_{1-4}$ alkyl group;
$R^{10}$ and $R^{11}$ each independently represent (1) a hydrogen atom or (2) a $C_{1-4}$ alkyl group;
n represents an integer from 0 to 4;
m represents an integer from 0 to 2; and
when n is two or more, the $R^1$' s may be the same or may differ from one another.)
or a salt thereof.

**8.** The agent according to any one of claims 1 to 6, wherein the compound having a Btk inhibitory activity is at least one selected from the group consisting of tirabrutinib, ibrutinib, spebrutinib, acalabrutinib, evobrutinib, poseltinib, fenebrutinib, vecabrutinib, zanubrutinib, PRN-1008, BMS-986142, and salts thereof.

**9.** The agent according to any one of claims 1 to 8, wherein the compound having a Btk inhibitory activity is tirabrutinib or a salt thereof.

**10.** Use of a compound having a Btk inhibitory activity to produce a preventive and/or therapeutic agent for an autoimmune disease selected from the group consisting of pemphigus, pemphigoid, ANCA-related angiitis, IgG4-related disease, nephrotic syndrome, and cutaneous lupus erythematosus.

**11.** A method for preventing and/or treating an autoimmune disease selected from the group consisting of pemphigus, pemphigoid, ANCA-related angiitis, IgG4-related disease, nephrotic syndrome, and cutaneous lupus erythematosus, including administering an effective amount of a compound having a Btk inhibitory activity to a mammal.

12. A compound having a Btk inhibitory activity, for use in the prevention and/or treatment of an autoimmune disease selected from the group consisting of pemphigus, pemphigoid, ANCA-related angiitis, IgG4-related disease, nephrotic syndrome, and cutaneous lupus erythematosus.

**INTERNATIONAL SEARCH REPORT**

International application No.
PCT/JP2019/018054

A. CLASSIFICATION OF SUBJECT MATTER
Int. Cl. [see extra sheet]

According to International Patent Classification (IPC) or to both national classification and IPC

B. FIELDS SEARCHED

Minimum documentation searched (classification system followed by classification symbols)
Int. Cl. A61K45/00, A61K31/4985, A61K31/505, A61K31/506, A61K31/517, A61K31/519, A61K31/52, A61K31/522, A61P9/00, A61P13/12, A61P17/00, A61P37/06, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | LEE, A. et al., Successful use of Bruton's kinase inhibitor, ibrutinib, to control paraneoplastic pemphigus in a patient with paraneoplastic autoimmune multiorgan syndrome and chronic lymphocytic leukemia, Australasian Journal of Dermatology, 2017, 58, e240-e242, abstract | 1, 3-5, 10-12<br>1, 3-5, 10-12<br>2, 6-9 |
| X<br>Y<br>A | RANKIN, A. L. et al., Selective inhibition of BTK prevents murine lupus and antibody-mediated glomerulonephritis, J Immunol., 2013, 191, pp. 4540-4550, abstract | 1, 3-5, 10-12<br>1, 3-5, 10-12<br>2, 6-9 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17.05.2019 | 04.06.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/018054

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | KIM, Y. et al., HM71224, a selective Bruton's tyrosine kinase inhibitor, attenuates the development of murine lupus, Arthritis Research & Therapy, 2017, 19, 211, pp. 1-11, abstract | 1, 3-5, 10-12<br>1, 3-5, 10-12<br>2, 6-9 |
| Y<br>A | MURREL, D. et al., A pilot study of the efficacy of a bruton's tyrosine kinase inhibitor in the treatment of dogs with pemphigus foliaceus, Australasian Journal of Dermatology, 2017, 58(S1), p. 73, entire text | 1, 3-5, 10-12<br>2, 6-9 |
| Y<br>A | CHALMERS, S. A. et al., Highly selective inhibition of Breton's tyrosine kinase attenuates skin and brain disease in murine lupus, Arthritis Research & Therapy, 25 January 2018, 20, 10, pp. 1-11, abstract, background | 1, 3-5, 10-12<br>2, 6-9 |
| Y<br>A | WO 2011/152351 A1 (ONO PHARMACEUTICAL CO., LTD.) 08 December 2011, claims, examples, in particular, example 19 (2) & JP 2015-52000 A & JP 2016-185954 A & US 2013/0079327 A1, claims, examples, especially example 19 (2) & US 2013/0217880 A1 & US 2015/0094299 A1 & US 2017/0313705 A1 & US 2018/0170933 A1 & EP 2578585 A1 & EP 3112368 A1 | 1, 3-5, 10-12<br>2, 6-9 |
| Y<br>A | MUSUMECI, F, et al., Pyrrolo[2, 3-d]pyrimidines active as Btk inhibitors, Expert Opinion on Therapeutic Patents, 2017, 27(12), pp. 1305-1318, abstract, fig. 3, 5-7 | 1, 3-5, 10-12<br>2, 6-9 |
| A | 森尾友宏, 好中球過剰活性化制御機構と炎症, 炎症と免疫, 2013, vol. 21, no. 4, pp. 345-351, non -official translation (MORIO, Tomohiro. Neutrophil Overactivation Control Mechanisms and Inflammation. Inflammation & Immunity.) | 1-12 |
| A | 石津明洋, 好中球細胞外トラップの異常とMPO-ANCA関連血管炎, 日本小児腎臓病学会雑誌, 2014, vol. 27, no. 2, pp. 81-85 (ISHIZU , Akihiro. Disorder of neutrophil extracellular traps in MPO-ANCA-associated vasculitis. Japanese Journal of Pediatric Nephrology.) | 1-12 |

Form PCT/ISA/210 (continuation of second sheet) (January 2015)

**INTERNATIONAL SEARCH REPORT**

International application No.
PCT/JP2019/018054

C (Continuation). DOCUMENTS CONSIDERED TO BE RELEVANT

| Category* | Citation of document, with indication, where appropriate, of the relevant passages | Relevant to claim No. |
|---|---|---|
| A | 石津明洋, 抗好中球細胞質抗体と好中球細胞外トラップ, 医学のあゆみ, 19 March 2016, vol. 256, no. 12, pp. 1209-1213, non -official translation (ISHIZU, Akihiro. Antineutrophil Cytoplasmic Antibodies and Neutrophil Extracellular Traps. Journal of Clinical and Experimental Medicine (IGAKU NO AYUMI).) | 1-12 |

Form PCT/ISA/210 (continuation of second sheet) (January 2015)

INTERNATIONAL SEARCH REPORT

International application No.
PCT/JP2019/018054

(continuation of Box A)

A61K45/00(2006.01)i, A61K31/4985(2006.01)i, A61K31/505(2006.01)i, A61K31/506(2006.01)i, A61K31/517(2006.01)i, A61K31/519(2006.01)i, A61K31/52(2006.01)i, A61K31/522(2006.01)i, A61P9/00(2006.01)i, A61P13/12(2006.01)i, A6G1P17/00(2006.01)i, A61P37/06(2006.01)i, A61P43/00(2006.01)i

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011152351 A
- WO 2013081016 A
- WO 2008039218 A
- WO 2008121742 A
- WO 2013010868 A
- WO 2012170976 A
- WO 2013067274 A
- WO 2011162515 A
- WO 2009158571 A
- WO 2013185084 A
- WO 2014173289 A
- WO 2014039899 A
- WO 2014210085 A
- WO 2016065226 A


### Non-patent literature cited in the description


- *Japanese Journal of Pediatric Nephrology,* 2014, vol. 27 (2), 11-15
- *CHEMICAL ABSTRACTS,* 936563-96-1
- *CHEMICAL ABSTRACTS,* 1420477-60-6
- *CHEMICAL ABSTRACTS,* 1415823-73-2
- *CHEMICAL ABSTRACTS,* 1434048-34-6
- *CHEMICAL ABSTRACTS,* 1353552-97-2
- *CHEMICAL ABSTRACTS,* 1202757-89-8
- *CHEMICAL ABSTRACTS,* 1510829-06-7
- *CHEMICAL ABSTRACTS,* 1691249-45-2
- *J. Immunol.,* 2009, vol. 183, 3980-3988
- *J. Immunol.,* 2013, vol. 191, 4540-4550